# EUROPEAN PATENT APPLICATION

(11) **EP 3 100 673 A1**
(43) Date of publication of application: **07.12.2016**
(21) Application number: 15170224.8
(22) Date of filing: 02.06.2015
(51) Int. Cl.: A61B 5/00, A61B 5/0402, A61B 5/113

(54) **BAND-TYPE HEART ELECTROMAGNETIC WAVE COLLECTING DEVICE AND METHOD FOR MANUFACTORING THE SAME**

(71) Applicant: Joint Concept (Hong Kong) Limited, New Territories (HK)
(72) Inventor: Wong, Kok Lap Victor, Hong Kong (HK)
(74) Representative: Casalonga

(57) **Abstract**

The present invention relates to a band-type heart electromagnetic wave collecting device, which includes a banded base and a waterproofing conductive electromagnetic wave collection portion disposed thereon, so that the electromagnetic wave collection portion is aligned with the heart to measure electromagnetic wave signals sent by the heart after the band-type heart electromagnetic wave collecting device is surrounded a human chest. The present invention also discloses a manufacture method of a band-type heart electromagnetic wave collecting device. Since a deposition technology is employed in the present invention to deposit metal ions with small resistivity on the device and auxetic materials are used, the device can better cling to skins, so that the data collected is more accurate. A plastic gasket added ensures the intensity and rigidness of the entire band-type heart electromagnetic wave collecting device, so that the device cannot be deformed due to the data collection process.

## Description

### Technical Field of the Invention

The present invention relates to the field of heart detection, and more particularly, to a band-type heart electromagnetic wave collecting device capable of accurately collecting heart electromagnetic waves and a method for manufacturing the same.

### Background of the Invention

Environment and health issues are increasingly concerned by common people due to the high speed development of the modem society. The rapid increasing of the industry and the unceasing progress of the society drive the environments, food and water sources which the mankind depends on for living polluted by various pollution sources. Additionally, with the increasing social stress, many adults are in the state of sub-health for long-term. Moreover, change of present diet structure leads middle aged and elderly people take a leapt risk of suffering from heart disease. Therefore, a simple and feasible instrument that can measure heart electromagnetic waves more accurately becomes very popular.

For example, US 6,477,397 discloses an electrode structure for measuring a biosignal from a living body, which includes a contact surface for placement against the body, the contact surface being formed with a plurality of protrusions that define an upper level and a plurality of valleys that define a lower level, wherein the peak of the upper level and the lower level define wave fronts. According to this invention, the contact surface is designed into a concavo-convex structure, which detects the physiological data of the heart through detection waves after being contacted with the heart part of the body. The invention solves the problem of simply testing the heart data of the body; however, when a collector is wet through after absorbing the perspiration of the body while moving, short circuit is caused to the product, which disables the product. Moreover, the electrode structure is complicated, and the manufacture cost is high, which is not beneficial for large-scale popularization.

Moreover, a three-point heart electromagnetic wave collector generally used in hospital is very inconvenient for users since it cannot be fixed on the body and has more line circuits, can be only used in hospital or in the bed, and is not convenient to use outside; thus the accuracy and realness of the collection results are very poor.

Therefore, it is necessary to improve the technology in prior art so as to be capable of manufacturing a waterproofing conductive heart electromagnetic wave collecting device which has a simpler structure, has low cost, and can accurately collect signals under the present technical conditions, so as to be beneficial for the consumption of the general mass.

### Summary of the Invention

According to one aspect of the present invention, the object of the present is to overcome the foregoing inherent problems of the prior art and the problem of disabled collector caused by short circuit when the collector is wet through after absorbing the perspiration of the body while moving, and provide a heart electromagnetic wave collecting device which has a simpler structure, has low cost, can collect data more accurately, and is simple to operate.

Another object of the present is to provide a method for manufacturing a heart electromagnetic wave collecting device, through which a product obtained can accurately collect the heart data, and is simple to operate.

In order to realize the foregoing objects, and according to an aspect of the present invention, the following technical solution is employed.

A band-type heart electromagnetic wave collecting device includes a banded base and a waterproofing conductive electromagnetic wave collection portion disposed on the base, so that the electromagnetic wave collection portion is aligned with the heart to measure electromagnetic wave signals sent by the heart after the band-type heart electromagnetic wave collecting device is surrounded a human chest.

Further, the band-type heart electromagnetic wave collecting device of the present invention includes:
a first part in a banded shape and having elasticity, wherein the first end of the first part is provided with a connecting part; and
a second part in a banded shape, wherein the first end of the second part is fixedly connected to the second end of the first part, and the second end of the second part can be buckled together with the second end of the first part, and wherein the second part includes:
   a first layer made of auxetic materials; and
   a second layer made of auxetic materials and overlapped together with the first layer, wherein the electromagnetic wave collection portion is disposed on the second layer.

According to one embodiment of the band-type heart electromagnetic wave collecting device of the present invention, the electromagnetic wave collection portion includes a positive electrode and a negative electrode.

According to one embodiment of the band-type heart electromagnetic wave collecting device of the present invention, one, two, three or four electromagnetic wave collection portions are disposed.

According to one embodiment of the band-type heart electromagnetic wave collecting device of the present invention, the electrodes refer to metal ions deposited on the first layer through vapor deposition.

According to one embodiment of the band-type heart electromagnetic wave collecting device of the present invention, the metal includes gold, silver or copper.

According to one embodiment of the band-type heart electromagnetic wave collecting device of the present invention, the band-type heart electromagnetic wave collecting device further includes a gasket disposed on the second layer.

According to one embodiment of the band-type heart electromagnetic wave collecting device of the present invention, the gasket is made of plastics. Preferably, the gasket is provided with a zinc oxide nanometer band so as to continuously measure an amplitude of respiration variations.

According to one embodiment of the band-type heart electromagnetic wave collecting device of the present invention, the band-type heart electromagnetic wave collecting device further includes a fixed buckle disposed on the first part.

According to one embodiment of the band-type heart electromagnetic wave collecting device of the present invention, an isolation layer made of auxetic materials is further included between the electromagnetic wave collection portions of the band-type heart electromagnetic wave collecting device.

According to one embodiment of the band-type heart electromagnetic wave collecting device of the present invention, the auxetic materials are auxetic polymer materials.

According to one embodiment of the band-type heart electromagnetic wave collecting device of the present invention, the gasket is fixed on the band-type heart electromagnetic wave collecting device through screws.

According to another aspect of the present invention, the present invention also provides a manufacture method of a band-type heart electromagnetic wave collecting device, which includes:
a method for manufacturing a band-type heart electromagnetic wave collecting device, wherein the method comprises:
   selecting a proper material to manufacture a banded base and a waterproofing conductive electromagnetic wave collection portion; and
   disposing the electromagnetic wave collection portion on the base to manufacture the band-type heart electromagnetic wave collecting device.

According to one embodiment of the invention, the method for manufacturing the waterproofing conductive electromagnetic wave collection portion comprises the following steps:
selecting a proper material to manufacture a flaky bottom layer;
depositing metal ions on the flaky bottom layer completely by applying a physical vapor deposition technology;
covering a layer of thin film completely on any surface of the flaky bottom layer; and
making the thin film completely fused with the flaky bottom layer into a piece of composite material to manufacture the electromagnetic wave collection portion.

According to one embodiment of the thin film is made of high polymers.

According to one embodiment of the invention, the material is selected from the group consisting of fiber cloth, polymer material or leather.

According to one embodiment of the invention, the material is selected from the group consisting of fiber cloth, polymer material or leather.

Since a deposition technology is employed in the present invention to deposit metal ions with small resistivity on the device and auxetic materials are used, the device can better cling to skins, so that the data collected is more accurate.

A plastic gasket added in the device ensures the intensity and rigidness of the entire band-type heart electromagnetic wave collecting device, so that the device cannot be deformed due to the data collection process.

The band-type heart electromagnetic wave collecting device of the present invention has simple operation process, can collect data accurately, and is beneficial for large-scale popularization.

### Brief Description of the Drawings

To explain the specific structure of the present invention clearly, the present invention will be described in details hereinafter with reference to the accompany drawings, wherein:
Fig. 1 shows an exploded view of one embodiment of a band-type heart electromagnetic wave collecting device according to the present invention; and
Fig. 2 shows an entire structure schematic view of one embodiment of the band-type heart electromagnetic wave collecting device according to the present invention.

### Detailed Description of the Invention

The present invention will be described in details hereinafter with reference to the drawings and specific embodiments. The schematic embodiments and explanation of the present invention here are only for explanation of the present invention, but are not intended to limit the present invention.

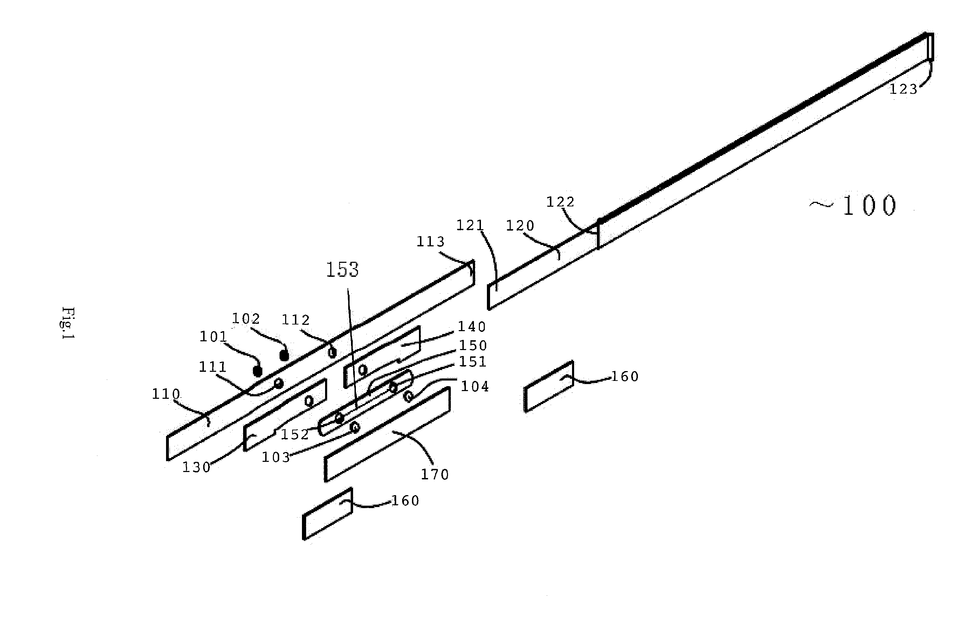

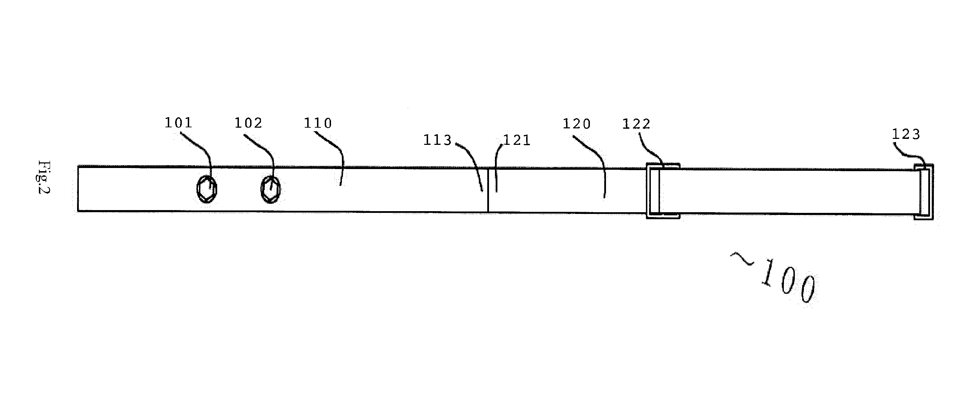

As shown in Fig. 1 and Fig. 2, a band-type heart electromagnetic wave collecting device 100 of the present invention includes a banded base and a waterproofing conductive electromagnetic wave collection portion 160 disposed on the base, so that the electromagnetic wave collection portion is aligned with the heart to measure electromagnetic wave signals sent by the heart after the band-type heart electromagnetic wave collecting device is surrounded a human chest.

The base includes a first part 120 in a banded shape and having elasticity. The first part 120 includes a first end 123, a second end 121 and an element 122 for adjusting the length of the entire first part 120. Through adjusting the element 122, the length of the entire first part 120 can be adjusted randomly, so as to adapt to people with different bodily forms, wherein the first end 123 includes a connecting portion.

The band-type heart electromagnetic wave collecting device 100 further includes a second part in a banded shape. The first end 123 is fixedly connected to the second end 121 of the first part 120, and the second end of the second part can be buckled together with the second end 121 of the first part 120, to form an annular band. The second part includes a first layer 110 which is made of auxetic materials, and further includes second layers 130 and 140 which are also made of auxetic materials, and are overlapped together with the first layer 110. Preferably, the auxetic materials are auxetic polymer materials.

The band-type heart electromagnetic wave collecting device 100 further includes the electromagnetic wave collection portion 160 which is disposed on the second layers 130 and 140, and jointed with the heart to collect the electromagnetic wave signals sent by the heart.

In one embodiment, the electromagnetic wave collection portion 160 includes two separate electrodes, i.e., a positive electrode and a negative electrode. Preferably, the electrodes are formed by metal ion electrodes, for example, gold ions, silver ions or copper ions or combinations thereof.

One, two, three or four electromagnetic wave collection portions 160 may be disposed according to the actual demands. Usually, two electromagnetic wave collection portions 160 are relatively rational. However, disposing three electromagnetic wave collection portions 160 is relatively rational for fat people, so as to be capable of accurately measuring the electromagnetic wave signals sent by the heart of the body.

The metal ion electrodes are deposited on the second layers 130 and 140 through a vapor deposition manner. The resistivity of the ion electrodes obtained through the vapor deposition manner is small, so that the data collected is more accurate. In one embodiment, the vapor deposition is physical vapor deposition so as to respectively and firmly fix the metal ions on the second layers 130 and 140.

The first layer 110 and the second layers 130 and 140 are fixed together. To be specific, holes 111 and 112 are disposed on the first layer 110; holes are respectively disposed on the second layers 130 and 140, and the two layers are combined together using screws 101 and 102.

Preferably, a gasket 150 is disposed on the second layer, for keeping the rigidness and intensity of the entire collection electrodes. Specifically, holes 151 and 152 are disposed on the gasket 150, and the gasket 150 is fixed on the second layers 130 and 140 through screws 103 and 104. In one embodiment, the gasket 150 is made of plastics, for example, polyethylene, polypropylene, polycarbonate, or ABS.

As shown in Fig. 1, the gasket 150 is provided with a zinc oxide nanometer band 153. The zinc oxide nanometer band 153 is embedded into the gasket 150, so as to continuously measure an amplitude of respiration variations. Since the self elasticity of the zinc oxide nanometer band 153 can better induce dilatation variations in a tiny range, the zinc oxide nanometer band is configured to detect the variations of a human lung during respiration when or after the moving of the body, so as to improve the detection precision of the entire device.

Further, an isolation layer 170 is also disposed between the electromagnetic wave collection portions 160, so as to leave a proper distance between the two electrodes, ensure the data collection accuracy, and make the entire band-type heart electromagnetic wave collecting device 100 more beautiful.

When in use, the electromagnetic wave collection portions 160 of the band-type heart electromagnetic wave collecting device of the present invention 100 need to be aligned with the heart part of the body only, the two ends are then buckled together, the tightness thereof is adjusted, and electromagnetic wave collection portions 160 are ensured to be aligned with the heart all the time. After adjustment, the auxetic materials are tightened and loosened through continuous deep respiration; and in combination with a signal transceiver and other adapted components, the electromagnetic wave signals sent by the heart can be exactly collected.

According to another aspect of the present invention, the present invention also provides a method for manufacturing a band-type heart electromagnetic wave collecting device, which includes:
selecting a proper material to manufacture a banded base and a waterproofing conductive electromagnetic wave collection portion; and
disposing the electromagnetic wave collection portion on the base to manufacture the band-type heart electromagnetic wave collecting device.

In one embodiment, the material can be selected from the group consisting of fiber cloth, polymer material or leather.

According to the method of the invention, the material can be selected from the group consisting of fiber cloth, polymer material or leather.

Further, a method for manufacturing the waterproofing conductive electromagnetic wave collection portion is as follows:
selecting a proper material to manufacture a flaky bottom layer;
depositing metal ions completely on the flaky bottom layer by applying a physical vapor deposition technology, wherein after the physical vapor deposition technology is applied on the flaky bottom layer, water molecules can still traverse freely, so that the flaky bottom layer is applied to detection after sweating;
covering a layer of thin film completely on any surface of the flaky bottom layer; and
making the thin film completely fused with the flaky bottom layer into a piece of composite material to manufacture the electromagnetic wave collection portion.

In one embodiment, the thin film is completely fused with the flaky bottom layer into a piece of composite material using a higher harmonic fusion technology. For example, a TM-001 vertical single-gluer laminator commercially available is employed to completely fuse the polymer thin film with the flaky bottom layer into a piece of composite material.

The manufactured composite material is cut into proper sizes according to the demand of the base, and then fixed on the base. The cutting is completed using a laser cutting or thermal cutting manner, for example, a JY-SL series laser cutting machine commercially available is employed to cut the base.

In one embodiment, the thin film is made of high polymers. In another embodiment, the material can be selected from fiber cloth, polymer material or leather.

In one embodiment, the base is made of textile materials, and the base is divided into two parts, i.e., a first part and a second part. The first part can be shaped as a band. The second part is made of auxetic materials, for example, auxetic polymer materials are employed to manufacture the base part having preferable elasticity.

In another embodiment, the electromagnetic wave collection portion is disposed on the second part. Preferably, the two are fixed using a fastening element (for example, a fastener).

Further, in order to increase the electromagnetic wave collection effect, a hard element (for example, a plastic block) is disposed between the second part and the electromagnetic wave collection portion, to enhance the test effect.

To verify the test result of the band-type heart electromagnetic wave collecting device of the present invention, a comparison test as follows is conducted:
20-45 years old healthy adults (the test results of 5-8 persons are selected, and the average value thereof is taken). Under the same test conditions, the band-type heart electromagnetic wave collecting devices employed are: (1) band-type heart electromagnetic wave collecting device without any auxetic material added thereinto; and (2) band-type heart electromagnetic wave collecting device with auxetic materials added in one part thereof and without auxetic materials in another part, wherein the average values of the test results obtained using the above-mentioned device (1) through a test method well-known to those skilled in the art are respectively as follows:
   the result of the first part of the device: 14.46mmHg; and
   the result of the second part of the device: 14.27mmHg;
   while the average values of the test results obtained using the above-mentioned device (2) are respectively as follows:
      the result of the first part of the device (without auxetic materials): 12.53mmHg; and
   the result of the second part of the device (with auxetic materials added): 16.79mmHg.

The above test object of the invention is to prove the influences produced by the wave amplitude size of the chest and the irregular modality of the chest when a person respires.

It can be seen from the above results that in the case that no auxetic materials are added, the signal results collected by the upper and lower parts of the band-type heart electromagnetic wave collecting device are basically the same; while in the case that one part of the band-type heart electromagnetic wave collecting device is added with auxetic materials and another part thereof not added, the measured result of the side added with the auxetic materials is more than the measured result of the side without auxetic materials by about 4 mmHg. Apparently, the test result of the band-type heart electromagnetic wave collecting device of the present invention is obviously improved than that of a usual collecting device. Generally, conducting materials cannot cling to the pit of the stomach if independently relying on an elastic band to tighten the chest; therefore, instability appears in electrocardio adoption data. Tests prove that the precision of the adoption data is increased after the auxetic materials are added.

Since a deposition technology is employed in the present invention to deposit metal ions with small resistivity on the device and auxetic materials are used, the device can better cling to skins, so that the data collected is more accurate. Moreover, after sweating of the body due to movement, the electromagnetic wave collection portion of the product of the present invention can be still used, and such situations like short circuit and no electricity cannot occur, thereby greatly improving the application range of the product of the present invention.

The above is detailed description to the technical solution provided by the embodiments of the present invention. Specific cases are applied herein to clearly interpret and explain the present invention; however, the above explanations of the implementation manners are only applied to help understand the principle and structure of the present invention. Meanwhile, those having ordinary skills in the art may made other modifications and/or variation according to the technical solution of the present invention as well as the spirits and principle thereof. It should be understood that all the modifications and/or variation shall fall into the protection scope of the present invention.

## Claims

1. A band-type heart electromagnetic wave collecting device, comprising: a banded base and a waterproofing conductive electromagnetic wave collection portion disposed on the base, so that the electromagnetic wave collection portion is aligned with the heart to measure electromagnetic wave signals sent by the heart after the band-type heart electromagnetic wave collecting device is surrounded a human chest.

2. The band-type heart electromagnetic wave collecting device according to claim 1, wherein the banded base comprises:
a first part in a banded shape and having elasticity, wherein the first end of the first part is provided with a connecting part; and
a second part in a banded shape, the first end of the second part is fixedly connected to the second end of the first part, and the second end of the second part can be buckled together with the second end of the first part, and wherein
the second part comprises:
a first layer made of auxetic materials; and
a second layer made of auxetic materials and overlapped together with the first layer, wherein the electromagnetic wave collection portion is disposed on the second layer.

3. The band-type heart electromagnetic wave collecting device according to claim 1, wherein the electromagnetic wave collection portion comprises a positive electrode and a negative electrode which are separated.

4. The band-type heart electromagnetic wave collecting device according to claim 3, wherein the positive electrode and the negative electrode are deposited by metal ions on the first layer in a vapor deposition manner.

5. The band-type heart electromagnetic wave collecting device according to claim 2, wherein the band-type heart electromagnetic wave collecting device further comprises a gasket disposed on the second layer.

6. The band-type heart electromagnetic wave collecting device according to claim 5, wherein the gasket is made of plastics.

7. The band-type heart electromagnetic wave collecting device according to claim 5, wherein the gasket is provided with a zinc oxide nanometer band so as to continuously measure an amplitude of respiration variations.

8. The band-type heart electromagnetic wave collecting device according to claim 2, wherein the band-type heart electromagnetic wave collecting device further comprises a fixed buckle disposed on the first part.

9. The band-type heart electromagnetic wave collecting device according to claim 2, wherein an isolation layer made of auxetic materials is further comprised between the electromagnetic wave collection portions of the band-type heart electromagnetic wave collecting device.

10. The band-type heart electromagnetic wave collecting device according to claim 2, wherein one, two, three or four the electromagnetic wave collection portions are disposed.

11. The band-type heart electromagnetic wave collecting device according to claim 5, wherein the gasket is fixed on the band-type heart electromagnetic wave collecting device through screws.

12. A method for manufacturing a band-type heart electromagnetic wave collecting device, wherein the method comprises:
selecting a proper material to manufacture a banded base and a waterproofing conductive electromagnetic wave collection portion; and
disposing the electromagnetic wave collection portion on the base to manufacture the band-type heart electromagnetic wave collecting device.

13. The method according to claim 12, wherein the method for manufacturing the waterproofing conductive electromagnetic wave collection portion comprises the following steps:
selecting a proper material to manufacture a flaky bottom layer;
depositing metal ions on the flaky bottom layer completely by applying a physical vapor deposition technology;
covering a layer of thin film completely on any surface of the flaky bottom layer; and
making the thin film completely fused with the flaky bottom layer into a piece of composite material to manufacture the electromagnetic wave collection portion.

14. The method according to claim 13, wherein the thin film is made of high polymers.

15. The method according to claim 12, wherein the material is selected from the group consisting of fiber cloth, polymer material or leather.

16. The method according to claim 13, wherein the material is selected from the group consisting of fiber cloth, polymer material or leather.
